# EUROPEAN PATENT APPLICATION

(11) **EP 4 851 959 A2**
(43) Date of publication of application: **09.12.2026**
(21) Application number: 26201243.8
(22) Date of filing: 13.09.2021
(51) Int. Cl.: G06V 20/69

(54) **AUTOMATED ANEUPLOIDY SCREENING USING ARBITRATED ENSEMBLES**

(30) Priority: 11.09.2020 US 202063077405 P; 11.09.2020 US 202063077398 P
(62) Document number(s) of the earlier application(s) in accordance with Art. 76 EPC: 21867754.0 / 4 211 606
(71) Applicant: The Brigham & Women's Hospital, Inc., Boston, Massachusetts 02115 (US); The General Hospital Corporation, Boston, MA 02114 (US)
(72) Inventor: SHAFIEE, Hadi, Boston, Massachusetts 02115 (US); BORMANN, Charles, Winchester, Massachusetts 01890 (US); KANAKASABAPATHY, Manoj Kumar, Boston, Massachusetts 02115 (US); THIRUMALARAJU, Prudhvi, Watertown, Massachusetts 02472 (US)
(74) Representative: Stellbrink & Partner Patentanwälte mbB

(57) **Abstract**

Systems and methods are provided for fully automated screening for aneuploidy in a human embryo. An image of the embryo is obtained at an associated imager and provided to a neural network to generate a first clinical parameter. A set of at least one parameter representing one of biometric parameters of one of a patient receiving the embryo, an egg utilized to produce the human embryo, a sperm used to create the embryo, a sperm donor who provided sperm used to create the embryo, and an egg donor who provided the egg is retrieved, and a second clinical parameter is generated from the set of at least one parameter at a predictive model. A composite parameter, representing a likelihood of aneuploidy in the embryo, is generated from the first clinical parameter and the second clinical parameter.

## Description

### Related Applications

The present application claims priority to each of U.S. Provisional Patent Application Serial No. 63/077,405 filed September 11, 2020 entitled ARTIFICIAL INTELLIGENCE-ENABLED SYSTEM TO AID IN ANEUPLOIDY SCREENING OF PREIMPLANTATION EMBRYOS and U.S. Provisional Patent Application Serial No. 63/077,398 filed September 11, 2020 entitled ARTIFICIAL INTELLIGENCE-ENABLED SYSTEM FOR ALIGNMENT OF OOCYTES AND PREIMPLANTATION EMBRYOS FOR INTRACYTOPLASMIC SPERM INJECTION (ICSI) AND ASSISTED HATCHING (AH) PROCEDURES. The entire content of each of these applications is incorporated herein by reference in its entirety for all purposes.

### Statement on Government Rights

This invention was made with government support under one or more of grant numbers R01AI118502, R01AI138800, and R21HD092828, awarded by the National Institutes of Health. The government has certain rights in the invention.

### Technical Field

The present invention relates generally to the field of assisted fertility, and more particularly, to automated aneuploidy screening using arbitrated ensembles of predictive models.

### Background of the Invention

Infertility is an underestimated healthcare problem that affects over forty-eight million couples globally and is a cause of distress, depression, and discrimination. Although assisted reproductive technologies (ART) such as in-vitro fertilization (IVF) has alleviated the burden of infertility to an extent, it has been inefficient with an average success rate of approximately twenty-six percent reported in 2015 in the US. IVF remains as an expensive solution, with a cost between $7000 and $20,000 per ART cycle in the US, which is generally not covered by insurance. Further, many patients require multiple cycles of IVF to achieve pregnancy. Non-invasive selection of the top-quality embryo for transfer is one of the most important factors in achieving successful ART outcomes, yet this critical step remains a significant challenge.

Preimplantation genetic testing for aneuploidy (PGT-A) has become a widely utilized tool for screening embryos for transfer. There are limited studies showing the efficacy of this procedure in women under thirty-five years of age. Conversely, there are some reports demonstrating that younger patients have reduced cumulative pregnancy rates when utilizing PGT-A. These lower outcomes are likely due to inaccurate PGT-A results or due to the invasive procedures involved with embryo biopsy and cryopreservation.

### Summary of the Invention

In accordance with an aspect of the present invention, a method is provided for fully automated screening for aneuploidy in a human embryo. An image of the embryo is obtained at an associated imager and provided to a neural network to generate a first clinical parameter. A set of at least one parameter representing one of biometric parameters of one of a patient receiving the embryo, an egg utilized to produce the human embryo, a sperm used to create the embryo, a sperm donor who provided sperm used to create the embryo, and an egg donor who provided the egg is retrieved, and a second clinical parameter is generated from the set of at least one parameter at a predictive model. A composite parameter, representing a likelihood of aneuploidy in the embryo, is generated from the first clinical parameter and the second clinical parameter.

In accordance with another aspect of the present invention, a system is provided for fully automated screening for aneuploidy in a human embryo. The system includes a processor and a non-transitory computer readable medium storing instructions executable by the processor. The machine executable instructions include an imager interface configured to receive an image of the embryo from an associated imager and a neural network configured to generate a first clinical parameter from the image of the embryo. A predictive model is configured to generate a second clinical parameter from a set of at least one parameter representing one of a patient receiving the embryo, an oocyte fertilized to produce the human embryo, a sperm used to create the embryo, a sperm donor who provided the sperm used to create the embryo, and an egg donor who provided the oocyte from an associated memory. An arbitrator is configured to generate a composite parameter, representing a likelihood of aneuploidy in the embryo, from the first clinical parameter and the second clinical parameter.

In accordance with yet another aspect of the present invention, a method is provided for fully automated screening for aneuploidy in a human embryo. An image of the embryo is obtained at an associated imager and a first clinical parameter is generated from the image of the embryo at a convolutional neural network. A set of parameters is retrieved from an associated memory. The set of parameters included each of an age of the egg donor who provided the oocyte fertilized to produce the embryo, a value representing a quality of the sperm used to create the embryo, and a number of embryos that were normally fertilized from oocytes harvested with the oocyte fertilized to produce the embryo. A second clinical parameter at a first predictive model from a first subset of the set of parameters, and a third clinical parameter at a second predictive model from a second subset of the set of parameters. A composite parameter, representing a likelihood of aneuploidy in the embryo, is generated from the first clinical parameter, the second clinical parameter, and the third clinical parameter.

### Brief Description of the Drawings

The foregoing and other features of the present invention will become apparent to those skilled in the art to which the present invention relates upon reading the following description with reference to the accompanying drawings, in which:
FIG. 1 illustrates one example of a system for fully automated screening for aneuploidy in a human embryo;
FIG. 2 illustrates another example of a system for fully automated screening for aneuploidy in a human embryo;
FIG. 3 illustrates an example of a method for screening for aneuploidy in a human embryo;
FIG. 4 illustrates another example of a method for screening for aneuploidy in a human embryo; and
FIG. 5 is a schematic block diagram illustrating an exemplary system of hardware components capable of implementing examples of the systems and methods disclosed herein.

### Detailed Description

Emulating the skill of highly trained embryologists in efficient embryo assessment in a fully automated system is a major unmet challenge in all of the previous work done in embryo computer-aided assessment. Current computer vision methods for embryo assessment are semi-automated, limited to measuring specific parameters providing metrics that require further analysis by embryologists, and require strictly controlled imaging systems. Previous attempts in developing systems using machine-learning approaches have required intensive image preprocessing followed by human-directed segmentation of embryo features for classification. Owing to the dependency of machine-learning approaches on image processing and segmentation, such methods suffer from the same limitations as computer vision techniques.

Here, we overcome this challenge by employing a deep neural networks pretrained with a large set of images, paired with biometric information about the egg, sperm, and their respective donors, to screen embryos for aneuploidy. Unlike prior computer-aided algorithms used for embryo assessment, the systems and methods provided herein allows for automated screening for aneuploidy without any assistance by an embryologist. In one example, a convolutional neural network is applied to identify the shape, structure, and texture variations between morphologically complex embryos, while one or more other predictive models are employed to evaluate the biometric data. The system is resilient to changes in image illumination and quality due to data acquisition using multiple instruments. Further, since the additional predictive models can be trained independently of the neural network, the system is effectively modular, allowing for addition or substitution of models in response to new applications.

The phrase "continuous parameter" is used herein to distinguish numerical values from categorical values or classes, and should be ready to include both truly continuous data as well as data more traditionally referred to as discrete data.

As used herein, an "average" of a set of values is any measure of central tendency. For continuous parameters, this includes any of the median, arithmetic mean, and geometric mean of the values. For categorical parameters, the average is the mode of the set of values.

A "static observation," as used herein, is an image or group of images of an embryo that represent a single point in the development of the embryo. Where multiple images are used in a static observation, no discernible change in the structure and appearance of the embryo will have taken place between images. Data collected from a static observation represents morphological data for the embryo.

A "subset" of a set as used here, refers to a set containing some or all elements of the set. A "proper subset" of a set contains less than all of the elements of the set.

As used herein, an embryo is "euploid" when is has forty-six chromosomes. An embryo is "aneuploid" when it has a number of chromosomes that is different from the usual forty-six. The "ploidy status" of the embryo is the embryos status as either euploid or aneuploid, represented either categorically or as a probability.

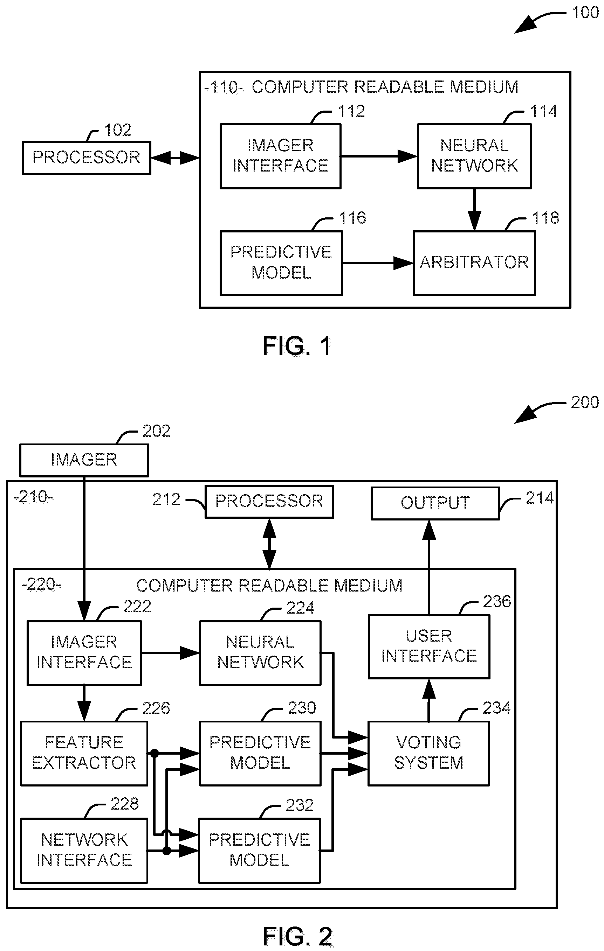

FIG. 1 illustrates one example of a system 100 for fully automated screening for aneuploidy in a human embryo. The system 100 includes a processor 102 and a non-transitory computer readable medium 110 that stores machine executable instructions for assigning a value representing a location of interest within a reproductive cellular structure. The machine executable instructions include an imager interface 112 that receives an image of embryo from an associated imager. For example, the imaging interface 112 can receive the image from the imager via a bus or network connection and condition the image for analysis at a neural network 114. In one example, the neural network 114 can be implemented on a cloud computing system, with the image transmitted to the server containing the neural network 114 via a network interface (not shown).

The neural network 114 determines, from the image of the reproductive cellular structure, a first clinical parameter representing a ploidy status of the embryo. In one implementation, the first clinical parameter is a categorical parameter with a first value representing aneuploidy and a second value representing euploidy. In another implementation, the first clinical parameter is a continuous parameter representing a likelihood of either aneuploidy or euploidy of the embryo. The neural network 114 includes a plurality of nodes having a plurality of interconnections. Values from the image, for example luminance and/or chrominance values associated with the individual pixels, are provided to a plurality of input nodes. The input nodes each provide these input values to layers of one or more intermediate nodes. A given intermediate node receives one or more output values from previous nodes. The received values are weighted according to a series of weights established during the training of the classifier. An intermediate node translates its received values into a single output according to an activation function at the node. For example, the intermediate node can sum the received values and subject the sum to an identify function, a step function, a sigmoid function, a hyperbolic tangent, a rectified linear unit, a leaky rectified linear unit, a parametric rectified linear unit, a Gaussian error linear unit, the softplus function, an exponential linear unit, a scaled exponential linear unit, a Gaussian function, a sigmoid linear unit, a growing cosine unit, the Heaviside function, and the mish function. A final layer of nodes provides the confidence values for the output classes of the neural network, with each node having an associated value representing a confidence for one of the associated output classes of the classifier.

Many ANN classifiers are fully-connected and feedforward. A convolutional neural network, however, includes convolutional layers in which nodes from a previous layer are only connected to a subset of the nodes in the convolutional layer. Recurrent neural networks are a class of neural networks in which connections between nodes form a directed graph along a temporal sequence. Unlike a feedforward network, recurrent neural networks can incorporate feedback from states caused by earlier inputs, such that an output of the recurrent neural network for a given input can be a function of not only the input but one or more previous inputs. As an example, Long Short-Term Memory (LSTM) networks are a modified version of recurrent neural networks, which makes it easier to remember past data in memory. In some examples, neural networks are trained in an adversarial manner in which a generative classifier is configured to generate new instances for a discriminative classifier. Typically, the generative network learns to map from a latent space to a data distribution of interest, while the discriminative network distinguishes candidates produced by the generator from a true data distribution. The generative network's training objective is to increase the error rate of the discriminative network. Each network learns iteratively to better model the true data distribution across the classes of interest.

The neural network 114 is trained on a plurality of labeled images of the appropriate reproductive cellular structure. By "labeled images," it is meant that the ploidy status of the embryo within the image is known, for example, via expert annotation, and the clinical parameter associated with ploidy status is provided to the neural network along with the image during the training process. During training, the weights associated with the interconnections among nodes in the neural network 114 are iteratively changed until, once the network is changed, an output of the network when presented with a novel, unlabeled image provides a clinical parameter representing the ploidy status of the embryo within the novel image. The first clinical parameter can be stored on the non-transitory computer readable medium 110.

A predictive model 116 is configured to generate a second clinical parameter from a set of at least one parameter representing one of a patient receiving the embryo, an egg utilized to produce the human embryo, a sperm used to create the embryo, a sperm donor who provided sperm used to create the embryo, and an egg donor who provided the egg. Examples of appropriate parameters include an age of the egg donor who provided the oocyte fertilized to produce the embryo, a value representing a quality of the sperm used to create the embryo, and a number of embryos that were normally fertilized from oocytes harvested with the oocyte fertilized to produce the embryo. Like the first clinical parameter, the second clinical parameter can be a categorical parameter with a first value representing aneuploidy and a second value representing euploidy or a continuous parameter representing a likelihood of either aneuploidy or euploidy of the embryo. It will be appreciated that the predictive model 116 can make use of additional biometric parameters representing of one of a patient receiving the embryo, a sperm donor who provided sperm used to create the embryo, an egg utilized to produce the human embryo, and an egg donor who provided the egg in determining a quality of the embryo as well as numerical features extracted from the image of the embryo. The second clinical parameter can be stored on the non-transitory computer readable medium 110

The predictive model 116 can utilize one or more pattern recognition algorithms, each of which analyze the extracted features or a subset of the extracted features to classify the patients into one of the plurality of classes and provide this information as a second clinical parameter. Where multiple classification or regression models are used, an arbitration element can be utilized to provide a coherent result from the plurality of models. The training process of a given classifier will vary with its implementation, but training generally involves a statistical aggregation of training data into one or more parameters associated with the output class. For rule-based models, such as decision trees, domain knowledge, for example, as provided by one or more human experts, can be used in place of or to supplement training data in selecting rules for classifying a patient using the extracted features. Any of a variety of techniques can be utilized for the classification algorithm, including support vector machines (SVMs), regression models, self-organized maps, fuzzy logic systems, data fusion processes, boosting and bagging methods, rule-based systems, or artificial neural networks.

For example, a support vector machine (SVM) classifier can utilize a plurality of functions, referred to as hyperplanes, to conceptually divide boundaries in the N-dimensional feature space, where each of the N dimensions represents one associated feature of the feature vector. The boundaries define a range of feature values associated with each class. Accordingly, an output class and an associated confidence value can be determined for a given input feature vector according to its position in feature space relative to the boundaries. In one implementation, the SVM can be implemented via a kernel method using a linear or non-linear kernel. The predictive model 116 can also use an artificial neural network classifier as described previously.

A rule-based classifier applies a set of logical rules to the extracted features to select an output class. Generally, the rules are applied in order, with the logical result at each step influencing the analysis at later steps. The specific rules and their sequence can be determined from any or all of training data, analogical reasoning from previous cases, or existing domain knowledge. One example of a rule-based classifier is a decision tree algorithm, in which the values of features in a feature set are compared to corresponding threshold in a hierarchical tree structure to select a class for the feature vector. A random forest classifier is a modification of the decision tree algorithm using a bootstrap aggregating, or "bagging" approach. In this approach, multiple decision trees are trained on random samples of the training set, and an average (*e.g.*, mean, median, or mode) result across the plurality of decision trees is returned. For a classification task, the result from each tree would be categorical, and thus a modal outcome can be used.

An arbitrator 118 is configured to generate a composite parameter, representing a likelihood of aneuploidy in the embryo, from the first clinical parameter and the second clinical parameter. In one implementation, the composite parameter is an average of a set of clinical parameters that includes the first clinical parameter and the second clinical parameter. In another implementation, the composite parameter is determined as a weighted linear combination of a set of clinical parameters that includes the first clinical parameter and the second clinical parameter. In this instance, the weights for each parameter can be determined according to a known accuracy of the neural network 114, the predictive model 116 and any additional models used to generate the parameters. In a still further implementation, the set of clinical parameters that includes the first clinical parameter and the second clinical parameter can be categorical, and the arbitrator 118 can select the composite parameter according to a voting algorithm. In one example, either a majority or plurality vote can be used. The composite parameter can then be stored on the non-transitory computer readable medium 110 and/or displayed to a user at an associated output device (not shown).

FIG. 2 illustrates another example of a system 200 for fully automated screening for aneuploidy in a human embryo. The system 200 includes an imager 202 that acquires one or more images of the embryo. For example, the imager 202 can include one or more cameras, capable of producing images in the visible or infrared range, paired with appropriate optics to provide an image of an embryo. In one implementation, the imager 202 can be implemented to capture images of an embryo at multiple days of development as part of a time-lapse embryo imaging system. In another implementation, the imager 202 can be configured to generate a static observation of the embryo as a set of one or more images. In one implementation, the imager 202 includes an attachment for a mobile device that operates with a camera of the mobile device to provide the embryo images. The housing for the attachment can be 3-D printed using polylactic acid with dimensions of 82 x 34 x 48 mm. An acrylic lens can be included in the housing to provided appropriate magnification for the embryo images.

In another implementation, the imager 202 can be implemented as a stand-alone system with an optical housing that is 3-D printed from polylactic acid and overall dimensions of 62 x 92 x 175 mm. The housing contains an electronic circuit with a white light-emitting diode, a three-volt battery, and a single pole double-throw switch. The embryo sample is transilluminated, with a 10x Plan-Achromatic objective lens for image magnification and a complementary metal-oxide-semiconductor (CMOS) image sensor for embryo image data acquisition. The CMOS sensor can be connected to a single-board computer to process the captured images. The imager 202 can be connected to a mobile device via a wireless connection (*e.g.*, Wi-Fi, Bluetooth, or a similar connection) for data processing and visualization.

Some or all of the one or more images obtained at the imager 202 are provided to an analysis system 210 comprising a processor 212, an output device 214, and a non-transitory computer readable medium 220 storing instructions executable by the processor. The instructions are executable to provide an imager interface 222 that receives the image or images of the embryo. The imager interface 222 can apply one or more imaging condition techniques, such as cropping and filtering, to better prepare the image for analysis. The images are then provided to a neural network 224 that provides a first clinical parameter representing the ploidy status of the embryo. In the illustrated implementation, the neural network 224 is trained on labeled images of euploid and aneuploid embryos, and provides a categorical output that can assume a first value, representing euploidy, and a second value, representing aneuploidy.

In one implementation, the neural network 224 can be a convolutional neural network, which is a feed-forward artificial neural network that includes convolutional layers, which effectively apply a convolution to the values at the preceding layer of the network to emphasize various sets of features within an image. In a convolutional layer, each neuron is connected only to a proper subset of the neurons in the preceding layer, referred to as the receptive field of the neuron. In one example, the convolutional neural network is implemented using the Xception architecture. In one implementation, at least one chromatic value (*e.g.*, a value for an RGB color channel, a YCrCb color channel, or a grayscale brightness) associated with each pixel is provided as an initial input to the convolutional neural network.

In another implementation, the neural network 224 can be implemented as a recurrent neural network. In a recurrent neural network, the connections between nodes in the network are selected to form a directed graph along a sequence, allowing it to exhibit dynamic temporal behavior. In another implementation, the neural network 224 is implemented and trained as a discriminative network in a generative adversarial model, in which a generative neural network and the discriminative network provide mutual feedback to one another, such that the generative neural network produces increasingly sophisticated samples for the discriminative network to attempt to classify. Regardless of the structure of the neural network 224, some or all layers of the neural network can be trained via transfer learning from another system, with only some of the layers trained on the training images of the reproductive cellular structure. A final layer of the neural network 224 can be implemented as a softmax layer to provide a classification result.

Some or all of one or more images can also be provided to a feature extractor 226 that reduces the image data into a feature vector comprising a plurality of values representing the content of the image or images. In particular, the feature extractor 226 extracts a plurality of features, which can be categorical, discrete, and continuous parameters representing the sensor data. It one example, the feature extractor 226 can utilize latent values from the neural network 224 as features representing the image or images. A network interface 228 can retrieve a set of biometric parameters from an associated memory, such as an electronic health records (EHR) database. The biometric parameters can represent one or more of a patient receiving the embryo, an egg utilized to produce the human embryo, a sperm used to create the embryo, a sperm donor who provided sperm used to create the embryo, and an egg donor who provided the egg from an associated memory. In the illustrated example, the parameters include an age of the egg donor who provided the oocyte fertilized to produce the embryo, a value representing a quality of the sperm used to create the embryo, and a number of embryos that were normally fertilized from oocytes harvested with the oocyte fertilized to produce the embryo.

A first subset of the values in the feature vector and the retrieved biometric parameters can be provided to a first predictive model 230 to generate a second clinical parameter. In the illustrated implementation, the first predictive model 230 is implemented as a support vector machine trained on labeled data representing euploid and aneuploid embryos, and provides the second clinical parameter as a categorical output that can assume a first value, representing euploidy, and a second value, representing aneuploidy. A second subset of the values in the feature vector and the retrieved biometric parameters can be provided to a second predictive model 232 to generate a third clinical parameter. In the illustrated implementation, the second predictive model 232 is implemented as a fully-connected feedforward neural network trained on labeled data representing euploid and aneuploid embryos, and provides the third clinical parameter as a categorical output that can assume a first value, representing euploidy, and a second value, representing aneuploidy.

The first, second, and third clinical parameters are provided to a voting system 234 that generates a composite parameter representing the ploidy status of the embryo. In the illustrated implementation, the composite parameter is generated according to a majority vote among the first, second, and third clinical parameters. The composite clinical parameter can be provided to a user at the output device 214 via a user interface 236. For example, the user interface 236 can include appropriate software instructions for receiving the output of the voting system 234 and presenting it at the output device 214. In one implementation, the output device 214 can include a mobile device that communicates wirelessly with the analysis system 210.

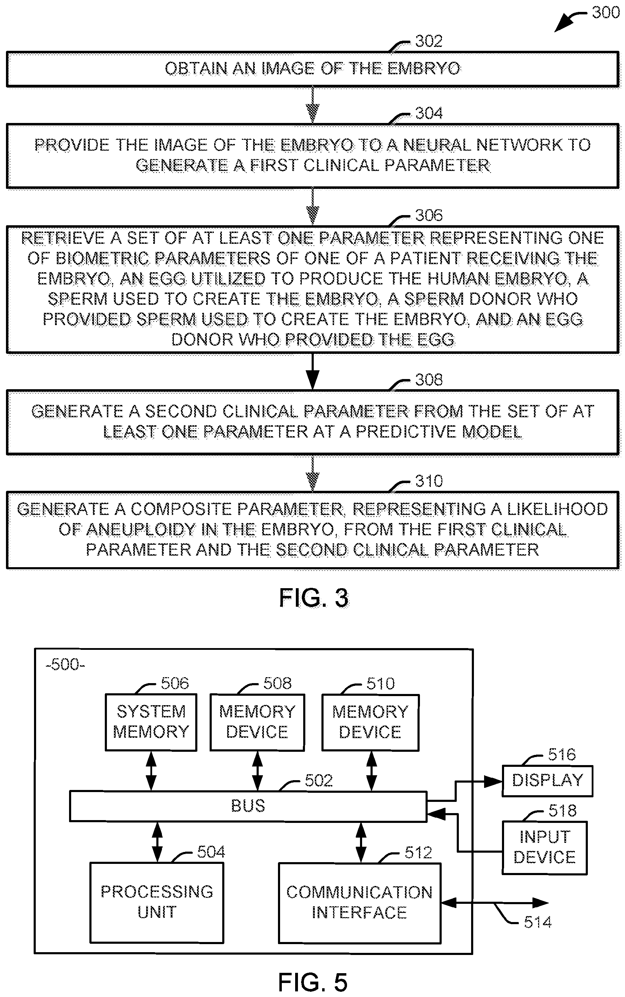

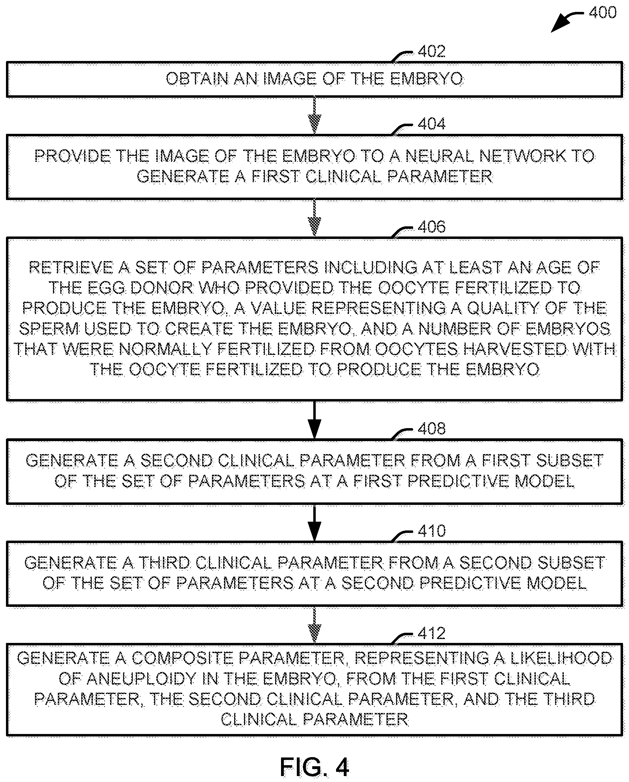

In view of the foregoing structural and functional features described above, a method in accordance with various aspects of the present invention will be better appreciated with reference to FIGS. 3 and 4. While, for purposes of simplicity of explanation, the methods of FIGS. 3 and 4 is shown and described as executing serially, it is to be understood and appreciated that the present invention is not limited by the illustrated order, as some aspects could, in accordance with the present invention, occur in different orders and/or concurrently with other aspects from that shown and described herein. Moreover, not all illustrated features may be required to implement a method in accordance with an aspect the present invention.

FIG. 3 illustrates an example of a method 300 for screening for aneuploidy in a human embryo. At 302, an image of the embryo is obtained at an associated imager. At 304, the image of the embryo to is provided a neural network to generate a first clinical parameter from the image of the embryo at a neural network. The first clinical parameter can be either categorical or continuous. The neural network can be implemented as any of a convolutional neural network, a recurrent neural network, and a discriminative classifier trained as part of a generative adversarial network. At 306, a set of at least one parameter representing one of biometric parameters of one of a patient receiving the embryo, an egg utilized to produce the human embryo, a sperm used to create the embryo, a sperm donor who provided sperm used to create the embryo, and an egg donor who provided the egg is retrieved. Examples of appropriate parameters include an age of the egg donor who provided the oocyte fertilized to produce the embryo, a value representing a quality of the sperm used to create the embryo, and a number of embryos that were normally fertilized from oocytes harvested with the oocyte fertilized to produce the embryo.

At 308, a second clinical parameter is generated from the set of at least one parameter at a predictive model. In one example, the predictive model is implemented as one of a support vector machine and a fully-connected feedforward neural network. At 310, a composite parameter, representing a likelihood of aneuploidy in the embryo, is generated from the first clinical parameter and the second clinical parameter. The composite parameter can be generated, for example, as an average of a set of clinical parameters that includes the first clinical parameter and the second clinical parameter, as a weighted linear combination of the set of clinical parameters, or according to a voting algorithm. The composite parameter can be stored in memory and/or displayed to a user at an associated display.

FIG. 4 illustrates another example of a method 400 for screening for aneuploidy in a human embryo. At 402, an image of the embryo is obtained. At 404, the image of the embryo to is provided a neural network to generate a first clinical parameter from the image of the embryo at a neural network. The first clinical parameter can be either categorical or continuous. The neural network can be implemented as any of a convolutional neural network, a recurrent neural network, and a discriminative classifier trained as part of a generative adversarial network. At 406, a set of parameters are retrieved from an associated memory. The set of parameters include at least an age of the egg donor who provided the oocyte fertilized to produce the embryo, a value representing a quality of the sperm used to create the embryo, and a number of embryos that were normally fertilized from oocytes harvested with the oocyte fertilized to produce the embryo.

At 408, a second clinical parameter is generated at a first predictive model from a first subset of the set of parameters. In one implementation, the first predictive model is implemented as a support vector machine. At 410, a third clinical parameter is generated at a second predictive model from a second subset of the set of parameters. In one implementation, the second predictive model is implemented as a fully-connected feedforward neural network. In one example, each of the first subset and the second subset contain all of the set of parameters, such that each of the first predictive model and the second predictive model receive the entire set of parameters. In another example, the first subset and the second subset are each proper subsets of the set of parameters. At 412, a composite parameter, representing a likelihood of aneuploidy in the embryo, is generated from the first clinical parameter, the second clinical parameter, and the third clinical parameter. In the illustrated example, the composite parameter is generated according to a majority vote among the first clinical parameter, the second clinical parameter, and the third clinical parameter. The composite parameter can be stored in memory and/or displayed to a user at an associated display.

FIG. 5 is a schematic block diagram illustrating an exemplary system 500 of hardware components capable of implementing examples of the systems and methods disclosed in FIGS. 1-4, such as the automated embryo evaluation system illustrated in FIG. 1. The system 500 can include various systems and subsystems. The system 500 can be any of personal computer, a laptop computer, a workstation, a computer system, an appliance, an application-specific integrated circuit (ASIC), a server, a server blade center, or a server farm.

The system 500 can includes a system bus 502, a processing unit 504, a system memory 506, memory devices 508 and 510, a communication interface 512 (e.g., a network interface), a communication link 514, a display 516 (e.g., a video screen), and an input device 518 (e.g., a keyboard and/or a mouse). The system bus 502 can be in communication with the processing unit 504 and the system memory 506. The additional memory devices 508 and 510, such as a hard disk drive, server, stand-alone database, or other non-volatile memory, can also be in communication with the system bus 502. The system bus 502 interconnects the processing unit 504, the memory devices 506-510, the communication interface 512, the display 516, and the input device 518. In some examples, the system bus 502 also interconnects an additional port (not shown), such as a universal serial bus (USB) port.

The system 500 could be implemented in a computing cloud. In such a situation, features of the system 500, such as the processing unit 504, the communication interface 512, and the memory devices 508 and 510 could be representative of a single instance of hardware or multiple instances of hardware with applications executing across the multiple of instances (i.e., distributed) of hardware (e.g., computers, routers, memory, processors, or a combination thereof). Alternatively, the system 500 could be implemented on a single dedicated server.

The processing unit 504 can be a computing device and can include an application-specific integrated circuit (ASIC). The processing unit 504 executes a set of instructions to implement the operations of examples disclosed herein. The processing unit can include a processing core.

The additional memory devices 506, 508, and 510 can store data, programs, instructions, database queries in text or compiled form, and any other information that can be needed to operate a computer. The memories 506, 508 and 510 can be implemented as computer-readable media (integrated or removable) such as a memory card, disk drive, compact disk (CD), or server accessible over a network. In certain examples, the memories 506, 508 and 510 can comprise text, images, video, and/or audio, portions of which can be available in formats comprehensible to human beings.

Additionally or alternatively, the system 500 can access an external data source or query source through the communication interface 512, which can communicate with the system bus 502 and the communication link 514.

In operation, the system 500 can be used to implement one or more parts of an embryo evaluation system in accordance with the present invention. Computer executable logic for implementing the composite applications testing system resides on one or more of the system memory 506, and the memory devices 508, 510 in accordance with certain examples. The processing unit 504 executes one or more computer executable instructions originating from the system memory 506 and the memory devices 508 and 510. It will be appreciated that a computer readable medium can include multiple computer readable media each operatively connected to the processing unit.

Specific details are given in the above description to provide a thorough understanding of the embodiments. However, it is understood that the embodiments can be practiced without these specific details. For example, circuits can be shown in block diagrams in order not to obscure the embodiments in unnecessary detail. In other instances, well-known circuits, processes, algorithms, structures, and techniques can be shown without unnecessary detail in order to avoid obscuring the embodiments.

Implementation of the techniques, blocks, steps, and means described above can be done in various ways. For example, these techniques, blocks, steps, and means can be implemented in hardware, software, or a combination thereof. For a hardware implementation, the processing units can be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described above, and/or a combination thereof.

Also, it is noted that the embodiments can be described as a process which is depicted as a flowchart, a flow diagram, a data flow diagram, a structure diagram, or a block diagram. Although a flowchart can describe the operations as a sequential process, many of the operations can be performed in parallel or concurrently. In addition, the order of the operations can be re-arranged. A process is terminated when its operations are completed, but could have additional steps not included in the figure. A process can correspond to a method, a function, a procedure, a subroutine, a subprogram, etc. When a process corresponds to a function, its termination corresponds to a return of the function to the calling function or the main function.

Furthermore, embodiments can be implemented by hardware, software, scripting languages, firmware, middleware, microcode, hardware description languages, and/or any combination thereof. When implemented in software, firmware, middleware, scripting language, and/or microcode, the program code or code segments to perform the necessary tasks can be stored in a machine readable medium such as a storage medium. A code segment or machine-executable instruction can represent a procedure, a function, a subprogram, a program, a routine, a subroutine, a module, a software package, a script, a class, or any combination of instructions, data structures, and/or program statements. A code segment can be coupled to another code segment or a hardware circuit by passing and/or receiving information, data, arguments, parameters, and/or memory contents. Information, arguments, parameters, data, etc. can be passed, forwarded, or transmitted via any suitable means including memory sharing, message passing, ticket passing, network transmission, etc.

For a firmware and/or software implementation, the methodologies can be implemented with modules (e.g., procedures, functions, and so on) that perform the functions described herein. Any machine-readable medium tangibly embodying instructions can be used in implementing the methodologies described herein. For example, software codes can be stored in a memory. Memory can be implemented within the processor or external to the processor. As used herein the term "memory" refers to any type of long term, short term, volatile, nonvolatile, or other storage medium and is not to be limited to any particular type of memory or number of memories, or type of media upon which memory is stored.

Moreover, as disclosed herein, the term "storage medium" can represent one or more memories for storing data, including read only memory (ROM), random access memory (RAM), magnetic RAM, core memory, magnetic disk storage mediums, optical storage mediums, flash memory devices and/or other machine readable mediums for storing information. The terms "computer readable medium" and "machine readable medium" includes, but is not limited to portable or fixed storage devices, optical storage devices, wireless channels, and/or various other storage mediums capable of storing that contain or carry instruction(s) and/or data. It will be appreciated that a "computer readable medium" or "machine readable medium" can include multiple media each operatively connected to a processing unit.

While the principles of the disclosure have been described above in connection with specific apparatuses and methods, it is to be clearly understood that this description is made only by way of example and not as limitation on the scope of the disclosure.

The invention is also defined by the following numbered aspects:
1. A method for fully automated screening for aneuploidy in a human embryo, comprising:
   obtaining an image of the embryo at an associated imager;
   providing the image of the embryo to a neural network to generate a first clinical parameter;
   retrieving a set of at least one parameter representing one of a patient receiving the embryo, an egg utilized to produce the human embryo, a sperm used to create the embryo, a sperm donor who provided sperm used to create the embryo, and an egg donor who provided the egg;
   generating a second clinical parameter from the set of at least one parameter at a predictive model; and
   generating a composite parameter, representing a likelihood of aneuploidy in the embryo, from the first clinical parameter and the second clinical parameter.
2. The method of aspect 1, wherein providing the image of the embryo to the neural network comprises providing the image of the embryo to a recurrent neural network.
3. The method of aspect 1, wherein providing the image of the embryo to the neural network comprises providing the image of the embryo to a discriminative classifier trained as part of a generative adversarial network.
4. The method of aspect 1, wherein providing the image of the embryo to the neural network comprises providing the image of the embryo to a convolutional neural network.
5. The method of aspect 1, wherein the set of at least one parameter includes an age of the egg donor who provided the oocyte fertilized to produce the embryo.
6. The method of aspect 1, wherein the set of at least one parameter includes a number of embryos that were normally fertilized from oocytes harvested with the oocyte fertilized to produce the embryo.
7. The method of aspect 1, wherein the set of at least one parameter includes a value representing a quality of the sperm used to fertilize the embryo.
8. A system for fully automated screening for aneuploidy in a human embryo, the system comprising:
   a processor; and
   a non-transitory computer readable medium storing instructions executable by the processor, the machine executable instructions comprising:
      an imager interface configured to receive an image of the embryo from an associated imager;
      a neural network configured to generate a first clinical parameter from the image of the embryo;
      a predictive model configured to generate a second clinical parameter from a set of at least one parameter representing one of a patient receiving the embryo, an oocyte fertilized to produce the human embryo, a sperm used to create the embryo, a sperm donor who provided the sperm used to create the embryo, and an egg donor who provided the oocyte from an associated memory; and
      an arbitrator configured to generate a composite parameter, representing a likelihood of aneuploidy in the embryo, from the first clinical parameter and the second clinical parameter.
9. The system of aspect 8, further comprising a feature extractor that generates a feature vector representing the image and provides the feature vector to the predictive model, the a predictive model configured to generate the second clinical parameter from at least one parameter representing the set of at least one parameter.
10. The system of aspect 8, wherein the arbitrator is configured to generate the composite parameter as a weighted linear combination of at least the first clinical parameter and the second clinical parameter.
11. The system of aspect 8, wherein the arbitrator is configured to generate the composite parameter as an average of at least the first clinical parameter and the second clinical parameter.
12. The system of aspect 8, wherein the predictive model is a first predictive model, the set of at least one parameter is a first set of at least one parameter, and the system further comprises a second predictive model configured to generate a third clinical parameter from a second set of at least one parameter, each of the first clinical parameter, the second clinical parameter, and the third clinical parameter being a categorical parameter and the arbitrator being configured to generate the composite parameter according to a majority vote among at least the first clinical parameter, the second clinical parameter, and the third clinical parameter.
13. The system of aspect 8, wherein the predicted model is implemented as a support vector machine.
14. The system of aspect 8, wherein the predicted model is implemented as a fully-connected feed-forward neural network.
15. The system of aspect 8, wherein the set of at least one parameter includes at least one of an age of the egg, an age of an egg donor, an age of a sperm donor, a method of fertilization for the embryo, a hormonal profile of the egg donor, a past diagnosis of a condition of the egg donor, and a past diagnosis of a condition of the sperm donor.
16. A method for fully automated screening for aneuploidy in a human embryo, comprising:
   obtaining an image of the embryo at an associated imager;
   generating a first clinical parameter from the image of the embryo at a convolutional neural network;
   retrieving a set of parameters from an associated memory, the set of parameters comprising an age of the egg donor who provided the oocyte fertilized to produce the embryo, a value representing a quality of the sperm used to create the embryo, and a number of embryos that were normally fertilized from oocytes harvested with the oocyte fertilized to produce the embryo;
   generating a second clinical parameter at a first predictive model from a first subset of the set of parameters;
   generating a third clinical parameter at a second predictive model from a second subset of the set of parameters; and
   generating a composite parameter, representing a likelihood of aneuploidy in the embryo, from the first clinical parameter, the second clinical parameter, and the third clinical parameter.
17. The method of aspect 16, wherein generating the composite parameter from the first clinical parameter, the second clinical parameter, and the third clinical parameter comprises generating the composite parameter according to a majority vote among the first clinical parameter, the second clinical parameter, and the third clinical parameter.
18. The method of aspect 16, wherein the first predictive model is implemented as a support vector machine, and the second predictive model is implemented as a fully-connected feedforward neural network.
19. The method of aspect 16, wherein generating the second clinical parameter at the first predictive model from the first subset of the set of parameters comprises generating the second clinical parameter at the first predictive model from the set of parameters, and generating the third clinical parameter at the second predictive model from the second subset of the set of parameters comprises generating the third clinical parameter at the second predictive model from the set of parameters.
20. The method of aspect 16, wherein each of the first subset of the set of parameters and the second subset of the set of parameters are proper subsets of the set of parameters.

## Claims

1. A method for fully automated screening for aneuploidy in a human embryo, comprising:
obtaining an image of the embryo at an associated imager;
providing the image of the embryo to a neural network to generate a first clinical parameter;
reducing image data from the image into a feature vector comprising a plurality of values representing the content of the image;
retrieving a set of biometric parameters, each representing one of a patient receiving the embryo, an egg utilized to produce the human embryo, a sperm used to create the embryo, a sperm donor who provided sperm used to create the embryo, and an egg donor who provided the egg;
providing a first subset of the plurality of values in the feature vector and the retrieved set of biometric parameters to a first predictive model to generate a second clinical parameter; and
generating a composite parameter, representing a likelihood of aneuploidy in the embryo, from the first clinical parameter and the second clinical parameter via an arbitrator.

2. The method according to claim 1, wherein reducing the image data into the feature vector comprises utilizing latent values from the neural network.

3. The method according to claim 1 or 2, further comprising providing a second subset of the values in the feature vector and the retrieved set of biometric parameters to a second predictive model to generate a third clinical parameter.

4. The method according to claim 3, wherein generating the composite parameter comprises generating the composite parameter according to a majority vote among the first clinical parameter, the second clinical parameter, and the third clinical parameter.

5. The method according to claim 3 or 4, wherein the second predictive model is a fully-connected feed-forward neural network.

6. The method according to any one of claims 1 to 5, wherein the neural network is a convolutional neural network.

7. The method according to any one of claims 1 to 5, wherein the neural network is implemented as a recurrent neural network.

8. The method according to any one of claims 1 to 7, wherein obtaining the image of the embryo at the associated imager comprises capturing images of the embryo as part of a time-lapse embryo imaging system.

9. A system comprising one or more processors and memory operably coupled with the one or more processors, wherein the memory stores instructions that, in response to execution of the instructions by the one or more processors, cause the one or more processors to perform the following operations:
obtaining an image of an embryo at an associated imager;
providing the image of the embryo to a neural network to generate a first clinical parameter;
reducing image data from the image into a feature vector comprising a plurality of values representing the content of the image;
retrieving a set of biometric parameters, each representing one of a patient receiving the embryo, an egg utilized to produce the embryo, a sperm used to create the embryo, a sperm donor who provided sperm used to create the embryo, and an egg donor who provided the egg;
providing a first subset of the plurality of values in the feature vector and the retrieved set of biometric parameters to a first predictive model to generate a second clinical parameter; and
generating a composite parameter, representing a likelihood of aneuploidy in the embryo, from the first clinical parameter and the second clinical parameter via an arbitrator.

10. The system according to claim 9, wherein reducing the image data into the feature vector comprises utilizing latent values from the neural network.

11. The system according to claim 9 or 10, wherein the operations further comprise providing a second subset of the values in the feature vector and the retrieved set of biometric parameters to a second predictive model to generate a third clinical parameter.

12. The system according to claim 11, wherein generating the composite parameter comprises generating the composite parameter according to a majority vote among the first clinical parameter, the second clinical parameter, and the third clinical parameter.

13. The system according to claim 11 or 12, wherein the second predictive model is a fully-connected feed-forward neural network.

14. The system according to any one of claims 9 to 13, wherein the neural network is implemented as a recurrent neural network.

15. The system according to any one of claims 9 to 14, wherein obtaining the image of the embryo at the associated imager comprises capturing images of the embryo as part of a time-lapse embryo imaging system.
